(19) Europäisches Patentamt
European Patent Office
Office européen des brevets

(11) Veröffentlichungsnummer: **0 396 024 A2**

(12) # EUROPÄISCHE PATENTANMELDUNG

(21) Anmeldenummer: **90107976.4**

(22) Anmeldetag: **26.04.90**

(51) Int. Cl.5: **C07C 69/76, C07C 67/11, A61K 31/235, C07C 67/343, C07D 307/935**

(30) Priorität: **05.05.89 DE 3914735**

(43) Veröffentlichungstag der Anmeldung: **07.11.90 Patentblatt 90/45**

(84) Benannte Vertragsstaaten: **AT BE CH DE DK ES FR GB GR IT LI LU NL SE**

(71) Anmelder: **Grünenthal GmbH**
**Zieglerstrasse 6**
**D-5100 Aachen(DE)**

(72) Erfinder: **Böhlke, Horst, Dr.**
**Oststrasse 45**
**D-5190 Stolberg(DE)**
Erfinder: **Seipp, Ulrich, Dr.**
**Pfeilstrasse 5**
**D-5100 Aachen(DE)**
Erfinder: **Winter, Werner, Prof. Dr.**
**Birkenstrasse 5**
**D-5100 Aachen(DE)**

(54) **Neue Ester, diese enthaltende Arzneimittel und die Herstellung dieser Ester und Arzneimittel.**

(57) Es werden neue wirksame Ester der Formel

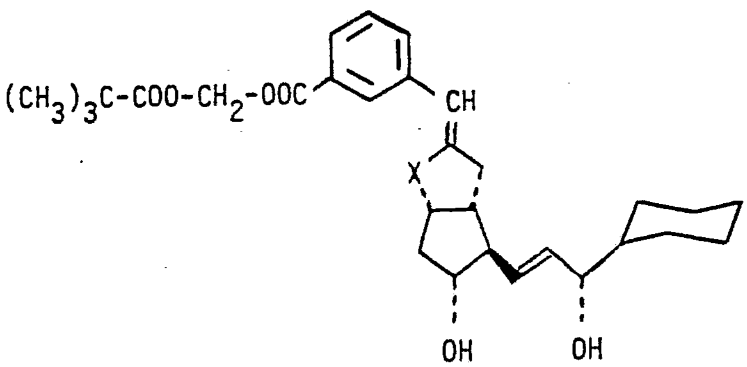

I

in der X für ein Sauerstoffatom oder für eine $CH_2$-Gruppe steht,
diese enthaltende Arzneimittel sowie die Herstellung dieser Ester und Arzneimittel beschrieben. Die Herstellung der pharmakologisch wirksamen Ester der Formel I kann z. B. durch Veresterungsreaktionen der diesen Estern zugrunde liegenden Säuren oder in Analogie zu den in den europäischen Patenten Nr. 45 842 bzw. Nr. 80 061 beschriebenen Verfahrensweisen erfolgen.

EP 0 396 024 A2

## Neue Ester, diese enthaltende Arzneimittel und die Herstellung dieser Ester und Arzneimittel

Aus der europäischen Patentschrift 45 842 und entsprechenden Schutzrechten in anderen Ländern sind z. B. Verbindungen der folgenden Formel A

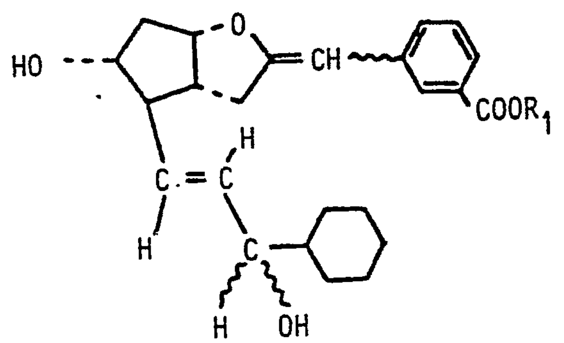

(A)

und aus der europäischen Patentschrift 80 061 und weiteren Schutzrechten Verbindungen der Formel B

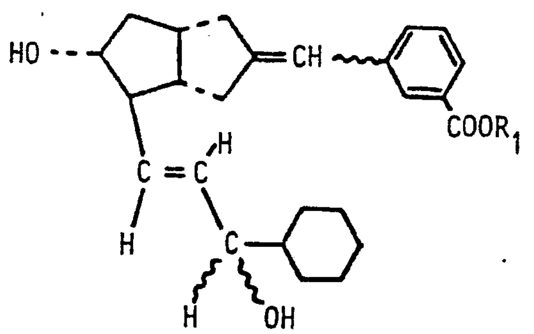

(B)

bekannt. In diesen Formeln A und B bedeutet $R_1$ ein Wasserstoffatom, ein pharmazeutisch verträgliches Kation oder einen geradkettigen oder verzweigten Alkylrest mit 1 - 6 bzw. 1 - 4 Kohlenstoffatomen.

Über die pharmakologischen Effekte der Verbindungen der Formel A (als Natriumsalz) und B (als freie Säure) wurde u. a. in Arzneimittelforschung/Drug Res. 33 (II) 1240 - 1248 (1983) berichtet. Über die biologische Wirkung von Estern dieser Verbindungen ist bisher nichts bekannt geworden.

In den meisten Literaturstellen, die sich auf pharmakologische Wirkungen von Prostacyclin- oder Carbacyclinderivaten beziehen, wurden die Verbindungen als freie Säuren bzw. deren Salze eingesetzt. Es finden sich nur relativ wenige Angaben über die biologische Aktivität von Estern. Während für bestimmte Prostaglandine bereits Acetoxymethyl-bzw. Acetoxyethylester beschrieben wurden, für die eine verbesserte Durchdringung von Zellmembranen angegeben wurde - vgl. EP 310 409 A2 -wurden diese oder vergleichbare Ester von Prostacyclinen oder Carbacyclinen noch nicht beschrieben. Aus den Angaben über Ester der letztgenannten Verbindungen ergibt sich, daß z. B. die Methylester der in Betracht gezogenen Prostacyclin- bzw. Carbacyclinderivate im Vergleich zu den entsprechenden freien Säuren ähnlich bzw. nur etwas weniger wirksam sind. Beispielsweise berichteten Fried und Barton in Proc. Natl. Acad. Sci. USA 74 - (1977) 2199 - 2203 über die Synthese des 13,14-Dehydroprostacyclinmethylesters und wiesen darauf hin, daß dieser Ester sich hinsichtlich der durch verschiedene Agentien bewirkten Aggregationshemmung von menschlichen Blutplättchen als ähnlich wirksam erwies wie natürliches Prostacyclin. In der britischen Patentanmeldung 2 017 699 A sind eine Anzahl von Carbacyclinderivaten beschrieben worden, von denen einige auch als Ester u. a. auf ihre Wirkung auf den Blutdruck von Hunden untersucht wurden. Dabei zeigte sich, daß der Methylester der (5EZ,13E)(9$\alpha$,11$\alpha$,15$\alpha$)-6,9-methano-11,15-dihydroxyprosta-5,13-diensäure etwa halb (ca. 50 - 60 %) so wirksam wie die freie Säure ist, während er hinsichtlich der Hemmung der durch ADP induzierten Blutplättchenaggregation in Rattenplasma etwa 80 % der Wirkung der freien Säure aufweist.

Gegenüber diesem Stand der Technik unerwartet zeigte z. B. der Methylester der Verbindung der Formel A bei intravenöser Verabreichung an Ratten erst in einer Dosis von mehr als dem 100fachen der

von der freien Säure benötigten Dosis eine blutdrucksenkende Wirkung, und auch für die Hemmung der durch ADP oder Arachidonsäure induzierten Plättchenaggregation war vom Methylester mehr als das 100fache der von der freien Säure benötigten Konzentration zur Erreichung der 50 %igen Hemmung erforderlich.

Diese Befunde zeigen, daß die Verbindungen der Formel A und B, in denen $R_1$ nicht für ein Wasserstoffatom oder ein Kation steht, als pharmakologisch praktisch unwirksam (weniger als 1 % der Wirkung der freien Säure aufweisend) anzusehen sind.

Es wurde nun überraschenderweise gefunden, daß die neuen Ester der Formel I

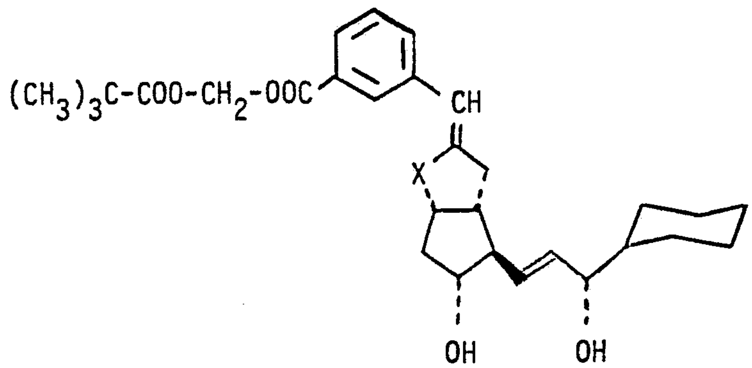

I

in der X für ein Sauerstoffatom oder für eine $CH_2$-Gruppe steht, sowohl in-vitro als auch in-vivo vergleichbare pharmakologische Wirkungen wie die freien Säuren aufweisen, gegenüber diesen aber eine verbesserte orale Absorption zeigen und sich insbesondere als lipidlösliche Komponenten zur Einarbeitung in Liposomen oder in injizierbare Lipidemulsionen eignen. Unter der Einwirkung von körpereigenen Enzymen werden die Ester der Formel I leicht zu den Verbindungen der Formel A bzw. Formel B hydrolysiert. Diese Hydrolyse ist ein wesentliches Merkmal der erfindungsgemäßen Ester. Es wird dazu angenommen, daß unspezifische Esterasen in einer ersten Stufe eine Hydrolyse zum entsprechenden Hydroxymethylester bewirken, der sich dann unmittelbar anschließen zu der freien Säureform (Formel A bzw. B) zersetzt.

Bei der oralen Verabreichung resultiert außerdem eine Verminderung von lokalen Unverträglichkeiten im Gastrointestinaltrakt, während bei der Verabreichung in Lipidformen weniger systemische Nebenwirkungen auftreten als nach Verabreichung der freien Säuren bzw. deren Salzen durch (zumeist mehrstündige) intravenöse Infusion. Darüber hinaus zeigte sich sowohl bei parenteraler als auch bei oraler Gabe der Ester der Formel eine ausgeprägt länger anhaltende Wirkung als bei analoger Verabreichung der freien Säuren.

So wurde an durch intraperitoneale Injektion von 60 mg/kg Natriumpentobarbital narkotisierten Ratten über einen Carotis-Katheter mit Hilfe eines Druckübermittlers der Blutdruck überwacht. Den Tieren (in Gruppen von je 4 Tieren) wurden dann für jeweils 10 Minuten über einen in die rechte vena femoralis eingesetzten Katheter

1) 10,0 µg/kg/min der Verbindung A ($R_1$ = H)

2) 9,6 µg/kg/min der Verbindung B ($R_1$ = H)

3) 6,2 µg/kg/min des Esters der Formel I (X = 0) bzw.

4) 10,5 µg/kg/min des Esters der Formel I (X = $CH_2$) injiziert, wobei diese Verbindungen in Form einer Lipidemulsion (vgl. Mizushima et al. J. Pharm. Pharmacol. 34 (1982) 49 - 50 und Ann. Rheum. Dis. 41 (1982) 263 - 267) zur Anwendung kamen. Dabei ergaben sich fol gende Werte des mittleren arteriellen Blutdrucks (in % des Ausgangswertes; jeweils als Mittelwert für die Tiere einer Gruppe):

| | Relativer Blutdruck (%) Minuten nach Injektionsbeginn | | |
|---|---|---|---|
| Tiergruppe | 5 | 10 | 20 |
| 1 | 64 | 65 | 94 |
| 2 | 80 | 84 | 105 |
| 3 | 58 | 62 | 81 |
| 4 | 62 | 51 | 69 |

Daraus ergibt sich, daß die den Tieren der Gruppen 3 und 4 verabreichten erfindungsgemäßen Ester stärker und länger andauernd den Blutdruck senkten als die jeweiligen ihnen zugrunde liegenden freien Säuren.

Zur Bestimmung der ADP-induzierten Thrombozytenaggregation an der Urethan-narkotisierten Ratte nach oraler Gabe der Vergleichssubstanzen wurde den Tieren ein Carotis-Katheter eingesetzt und über diesen kontinuierlich Blut entnommen. Nach Zumischen von Natriumzitrat wurde in einem Technicon-Thrombocounter die Thrombozytenzahl bestimmt. 15 Minuten und unmittelbar vor der Gabe der Prüfsubstanz sowie 15, 30, 45 und 60 min. nach der Prüfsubstanzgabe wurden über die vena jugularis jeweils 100 µg/kg ADP als Bolus injiziert und der Thrombozytenabfall unter der ADP-Injektion bestimmt. Der Mittelwert der beiden Werte vor der Prüfsubstanzgabe wurde als Ausgangs-Kontrollwert genommen und die Werte nach Prüfsubstanzgabe als %-Änderung gegenüber diesem Wert bestimmt.

| Substanz | Dosis (mg/kg) | Tierzahl der Versuchsgruppe | Thrombozytenaggregationshemmung in % des Ausgangskontrollwertes nach Minuten | | | |
|---|---|---|---|---|---|---|
| | | | 15 | 30 | 45 | 60 |
| B($R_1$ = H) | 10 | 8 | 68,9±6,8 | 55,1±6,7 | 51,8±6,4 | 38,1±6,3 |
| I(X = $CH_2$) | 10 | 9 | 24,0±3,0 | 28,1±3,8 | 29,1±3,9 | 30,3±4,6 |
| I(X = $CH_2$) | 100 | 3 | 59,4±3,0 | 63,3±6,8 | 68,1±2,0 | 71,0±4,4 |

Während also der Effekt bei beiden Dosen des Esters der Formel I (X = $CH_2$) über die Versuchszeit anhielt bzw. sich leicht verstärkte, ging der Effekt der Substanz B ($R_1$ = H) über die Versuchszeit, insbesondere aber ab der 45. Versuchsminute, deutlich zurück.

Wie bereits gesagt, weisen die Ester der Formel I auch bei Verabreichung an den Menschen bei guter Wirkung im Vergleich zu den freien Säuren verbesserte Eigenschaften auf. Die Erfindung betrifft dementsprechend auch Arzneimittel, die als Wirkstoff einen der erfindungsgemäßen Ester enthalten. Der Wirkstoffgehalt pro Einzeldosis beträgt dabei etwa 0,01 - 50 mg, und zwar bei Zubereitungsformen für die parenterale Applikation etwa 0,01 - 10 mg und bei Zubereitungen für die orale Applikation etwa 0,1 - 50 mg. Bei den Arzneimittelformen für die parenterale Anwendung handelt es sich bevorzugt um Mikrokapseln bzw. Liposomen, die einen Ester der Formel I in gelöster Form, z. B. in einem geeigneten Öl, enthalten, oder um Lipidemulsionen dieser Ester, wie z. B. die oben genannten.

Für die orale Verabreichung kommen vorzugsweise Kapseln in Betracht, die die Wirkstoffe gelöst in einem geeigneten, insbesondere einem verdaulichen Öl enthalten.

Für viele prophylaktische oder therapeutische Anwendungen kommen zweckmäßig auch perkutane Applikationszubereitungen (wie z. B. die Wirkstoffe in einem Depot in gelöster Form, gegebenenfalls unter Zusatz von die Hautpenetration fördernden Mitteln enthaltende Pflaster oder dergleichen) in Betracht.

Vorteilhaft werden die oral oder perkutan anwendbaren Zubereitungsformen so hergestellt, daß daraus der Wirkstoff verzögert freigesetzt wird, um so über einen längeren Zeitraum (beispielsweise 24 Stunden) eine gleichförmige Versorgung des Patienten mit dem Wirkstoff zu gewährleisten.

Als Indikationen kommen für die Ester der Formel I die gleichen in Betracht, wie die für die Verbindungen der Formeln A bzw. B z. B. in den einleitend zitierten Patenten genannten, d. h. sie eignen sich sowohl bei parenteraler als auch bei oraler Verabreichung zur Hemmung der Thrombozytenaggregation am Menschen, und zwar zur Vorbeugung und Behandlung von Krankheitszuständen, bei denen die Thrombozytenaggregation und/oder eine Hyperaggregabilität pathogenetisch von Bedeutung sind. Solche Krankheitsbilder sind z. B. arterielle Thrombosen bei Endotheläsionen, Atherosklerose, hämostatische arterielle und venöse Thrombosen sowie der Myokardinfakt. Aufgrund ihrer Wirkung auf den Blutdruck kommen die Ester der Formel I auch zur Behandlung von pulmonalem sowie systemischem Hochdruck in Betracht. Vorteilhaft sind die erfindungsgemäßen Substanzen auch zur Verminderung der Aggregabilität bei extrakorporalen Blutkreisläufen (künstliche Niere, Herz-Lungen-Maschine etc.) einsetzbar, wobei man die Substanzen in mikromolaren Konzentrationen dem Patientenblut zusetzt.

Die erfindungsgemäßen Ester bewirken ferner eine Verminderung der Magensäuresekretion. Sie kommen daher auch zur Behandlung von Erkrankungen mit erhöhter Magensäuresekretion wie z. B. Magen- und Darmulcera, außerdem aber auch zur Therapie von Ulcuserkrankungen anderer Genese, wie z. B. Antiphlogistica-ulcera, in Betracht.

Die erfindungsgemäße Herstellung dieser Arzneimittel erfolgt in an sich bekannter Weise, wobei selbstverständlich bei der Herstellung der parenteral anzuwendenden Zubereitungen auf Sterilität zu achten

ist.

Zur Herstellung der neuen Ester der Formel I kann man von einer Verbindung der Formel A oder B oder einem Salz einer solchen Verbindung ausgehen. Diese Ausgangsmaterialien werden zusammengefaßt nachstehend durch die Formel II wiedergegeben.

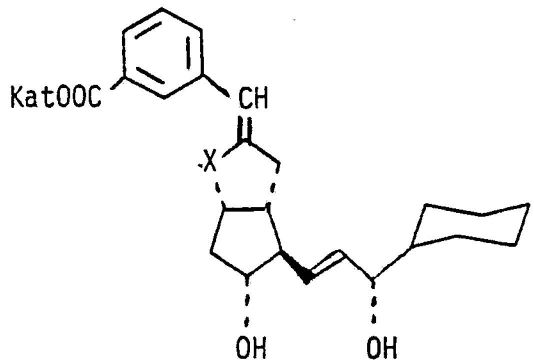

II

Darin bedeutet Kat ein Wasserstoffion oder ein Kation, vorzugsweise ein Alkalimetallion, und X hat die gleiche Bedeutung wie in Formel I. Kat kann ein anorganisches oder ein organisches Kation, wie z. B. das Triethylammoniumion, sein.

Durch Umsetzung einer Verbindung der Formel II mit einem Pivalinsäuremethylesterderivat der Formel III,

$(CH_3)_3C\text{-}COO\text{-}CH_2\text{-}Y$ III

worin Y ein Chlor-, Brom- oder Jodatom, vorzugsweise ein Chlor- oder Bromatom, oder eine Acyloxy-, Alkylsulfonyloxy- oder Arylsulfonyloxygruppe, insbesondere eine Methylsulfonyloxy- oder eine p-Tolylsulfonyloxygruppe ist,

gegebenenfalls unter Zusatz eines säurebindenden Stoffes, wie z. B. Natrium- oder Kaliumcarbonat und/oder unter Zusatz eines Alkalijodids, lassen sich die Verbindungen der Formel I in an sich bekannter Weise herstellen.

Die Umsetzung wird in einem inerten organischen Lösungsmittel wie Aceton, Dioxan, Tetrahydrofuran, Dimethylformamid oder Dichlormethan und bei Raumtemperatur oder auch bei erhöhten Temperaturen (bis etwa 75° C) durchgeführt. Gegebenenfalls können zuvor die Hydroxygruppen in der Verbindung der Formel II durch leicht - ohne Zerstörung der Estergruppierung in der angestrebten Verbindung der Formel I - wieder abspaltbare Gruppen intermediär geschützt werden. Solche Gruppen sind beispielsweise die Trimethylsilyl- oder die Dimethyl- oder Diphenyl-tert.butylsilylgruppe, die durch Hydrolyse unter neutralen oder schwach sauren Bedingungen selektiv nach der Veresterungsreaktion entfernbar sind.

Alternativ kann man auch so vorgehen, daß man eine Verbindung der Formel II, in der in diesem Fall Kat vorzugsweise ein Alkalimetallkation bedeutet, mit einer Verbindung der Formel IV $Cl\text{-}CH_2\text{-}Z$ (IV)

in der Z Brom, Jod oder einen Alkylsulfonyloxy- oder einen Arylsulfonyloxyrest, insbesondere einen Methylsulfonyloxy- oder p-Tolylsulfonyloxyrest, bedeutet

unter den gleichen Bedingungen, wie sie für die Umsetzung der Verbindungen der Formeln II und III genannt wurden, umsetzt und dann das so erhaltene Zwischenprodukt der Formel V

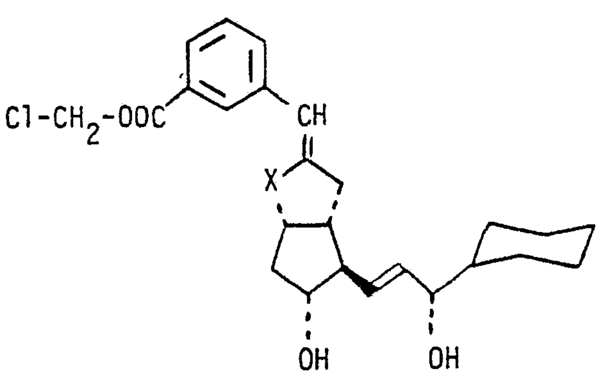

V

worin X die gleiche Bedeutung wie oben hat,

mit einem Salz der Pivalinsäure unter Bildung der Verbindung der Formel I zur Reaktion bringt. Die bei diesem letztgenannten Reaktionsschritt angewendeten Bedingungen entsprechen den für die Reaktion der Verbindungen der Formeln II und III vorstehend geschilderten.

Schließlich kann man zur Herstellung einer Verbindung der Formel I auch entsprechend den in den oben genannten europäischen Patenten Nr. 45 842 bzw. Nr. 80 061 genannten Verfahrensweisen vorgehen. So kann man z. B. ausgehend von 3-Methylbenzoesäure deren Pivaloyloxymethylester herstellen, darin die 3-Methylgruppe halogenieren und durch Behandlung mit Triphenylphosphin in das entsprechende Phosphoniumsalz überführen. Das daraus durch Behandeln mit einer starken Base, insbesondere Natrium-bis-(trimethylsilyl)-amid, erhaltene Phosphinalkylenderivat wird dann mit 6-$\beta$-(3′S-Hydroxy-3′-cyclohexyl-1′E-propenyl)-7$\alpha$-hydroxy-cis-bicyclo[3.3.0] octan-3-on umgesetzt und schließlich aufgearbeitet, wobei man die entsprechende Verbindung der Formel I erhält.

Die folgenden Beispiele dienen zur weiteren Erläuterung der Erfindung. Alle Temperaturangaben darin sind unkorrigiert.

Bei der Durchführung der Beispiele wurde auf die Erzielung maximaler Ausbeuten kein Wert gelegt.

Die $^1$H-NMR-Spektren wurden bei 300,13 MHz mit einem handelsüblichen Gerät aufgenommen. Die chemischen Verschiebungen sind in ppm angegeben. Die in Anführungszeichen gesetzten Multiplizitäten zeigen bei höherer Auflösung weitere Aufspaltungen.

## Beispiel 1

2-Oxa-3Z-(m-pivaloyloxymethyloxycarbonyl-benzyliden)-6$\beta$-(3′S-hydroxy-3′-cyclohexyl-1′E-propenyl)-7$\alpha$-hydroxy-cis-bicyclo[3.3.0]octan (= Verbindung der Formel I, in der X für ein Sauerstoffatom steht).

Zu 500 mg des Natriumsalzes von 2-Oxa-3Z-(m-carboxybenzyliden)-6$\beta$-(3′-S-hydroxy-3′cyclohexyl-1′E-propenyl)-7$\alpha$-hydroxy-cis-bicyclo[3.3.0] octan in 10 ml trockenem N,N-Dimethylformamid werden bei Raumtemperatur unter Feuchtigkeitsausschluß 0.5 ml Pivalinsäurechlormethylester gegeben. Das Reaktionsgemisch wird 18 Stunden gerührt, anschließend zu 30 ml Wasser gegeben und dreimal mit je 30 ml Essigester ausgeschüttelt. Die vereinigten Essigesterauszüge werden mit 10 ml gesättigter Kochsalzlösung gewaschen, über Magnesiumsulfat getrocknet und am Rotationsverdampfer bei 40° C/10 mbar eingeengt. Das Rohprodukt (900 mg) wird mittels Säulenchromatographie an Kieselgel mit Hexan-Essigester 1 : 4 gereinigt, wobei man nach Abdampfen der Lösungsmittel 500 mg der Titelverbindung erhält.

Zur weiteren Reinigung wird das Produkt in Essigester aufgenommen und durch Zugabe von Hexan ausgefällt. Ausbeute 330 mg (= 53 % der Theorie). Schmelzpunkt: 68 - 70° C. $^1$H-NMR ($CDCl_3$):

8,15 "t" 1H 5,28 "S" 1H 2,34 - 2,42 m 1H
7,82 - 7,84 "d" 1H 4,87 - 4,91 m 1H 2,14 - 2,24 m 1H
7,76 - 7,78 "d" 1H 3,90 - 3,99 "q" 1H 1,26 - 2,02 m 10H
7,32 - 7,37 "t" 1H 3,82 - 3,86 "t" 1H 1,23 s 9H
5,99 s 2H 2,90 - 2,98 m 1H 0,88 - 1,18 m 4H
5,46 - 5,67 m 2H 2,55 - 2,69 m 2H

## Beispiel 2

3E-(m-Pivaloyloxymethyloxycarbonyl-benzyl iden)-6$\beta$-(3′S-hydroxy-3′-cyclohexyl-1′E-propenyl)-7$\alpha$ hydroxy-cis-bicyclo[3.3.0]octan (= Verbindung der Formel I, in der X für eine $CH_2$-Gruppe steht).

Zu einer Lösung von 1,98 g 3E-(m-Carboxybenzyliden)-6$\beta$-(3′S-hydroxy-3′-cyclohexyl-1′E-propenyl)-7$\alpha$-hydroxy-cis-bicyclo-[3.3.0]octan in 50 ml wasserfreiem Aceton gibt man 1,04 g getrocknetes Kaliumcarbonat und rührt das Gemisch 1 Stunde bei Raumtemperatur. Danach gibt man eine Lösung von 0,8 ml Pivalinsäurechlormethylester in 10 ml wasserfreiem Aceton langsam tropfenweise zu und erhitzt die Mischung anschließend drei Tage unter Rühren und Feuchtigkeitsausschluß zum Rückfluß. Nach dem Abkühlen filtriert man vom Niederschlag ab, wäscht mit Aceton nach und engt das Filtrat am Rotationsverdampfer ein. Das zurückbleibende ölige Produkt wird mittels Säulenchromatographie an Kieselgel mit Essigsäureethylester gereinigt. Ausbeute: 1,4 g (= 54,8 % d. Th.) der Titelverbindung.

Das bei Raumtemperatur langsam kristallisierende Produkt läßt sich aus Diisopropylether umkristallisieren. Es zeigt dann einen Schmelzpunkt von 102 - 105,5° C. $^1$H-NMR ($CDCl_3$):

7,95 "s" 1H 2,68 - 2,57 m 2H
7,89 - 7,86 "d" 1H 2,41 - 2,13 m 5H
7,48 - 7,37 "d" + t 2H 1,94 - 1,85 m 2H

6,43 "s" 1H 1,75 - 1,66 m 5H
6,00 s 2H 1,44 - 1,36 m 1H
5,62 - 5,46 m 2H 1,30 - 1,12 m 3H
3,83 - 3,67 m 2H 1,23 s 9H
2,82 - 2,73 m 1H 1,03 - 0,91 m 3H

Beispiel 3

a) 3-Methyl-benzoesäure-pivaloyloxymethylester

Zu einer Lösung von 6,81 g 3-Methyl-benzoesäure in 500 ml wasserfreiem Aceton gibt man 10,37 g gepulvertes trockenes Kaliumcarbonat und rührt das Gemisch unter Feuchtigkeitsausschluß 1 Stunde bei Raumtemperatur. Danach gibt man tropfenweise die Lösung von 7,93 ml Pivalinsäurechlormethylester in 10 ml wasserfreiem Aceton langsam zu und erhitzt anschließend unter weiterem Rühren 30 Stunden zum Rückfluß. Nach dem Abkühlen saugt man vom festen Anteil ab, wäscht mit Aceton nach und engt das Filtrat am Rotationsverdampfer ein. Der ölige Rückstand wird durch Chromatographie an Kieselgel mit Diisopropylether gereinigt. Ausbeute: 10,15 g (= 81 % d. Th.). $^1$H-NMR ($CDCl_3$):
7,88 - 7,86 "s" + "d" 2H 2,41 s 3H
7,42 - 7,32 "d" + t 2H 1,23 s 9H
5,99 s 2H

b) 3-Brommethyl-benzoesäure-pivaloyloxymethylester

Zu einer Lösung von 6,41 g 3-Methyl-benzoesäure-pivaloyloxymethylester (erhalten in Beispiel 3a) in 50 ml Tetrachlorkohlenstoff gibt man 5,07 g N-Bromsuccinimid sowie 0,3 g Dibenzoylperoxid und erwärmt das Gemisch unter intensivem Rühren. Bei ca. 90° C Badtemperatur setzt die Reaktion unter Schäumen und Gasentwicklung ein. Man erhöht die Temperatur des Heizbades auf 100° C und erhitzt das Reaktionsgemisch noch 1 Stunde zum Rückfluß.

Nach dem Abkühlen filtriert man das gebildete Succinimid ab, wäscht mit Tetrachlorkohlenstoff nach und engt das Filtrat am Rotationsverdampfer ein. Der ölige Rückstand wird durch Chromatographie an Kieselgel mit Diisopropylether/Petrolether 1 : 20 gereinigt. Ausbeute: 6,38 g (= 76 % d. Th.). $^1$H-NMR ($CDCl_3$):
8,09 - 8,08 "s" + "d" 1H 6,00 s 2H
8,02 - 7,99 "d" 1H 4,52 s 2H
7,65 - 7,62 "d" 1H 1,23 s 9H
7,48 - 7,43 t 1H

c) 3-Triphenylphosphoniummethyl-benzoesäure-pivaloyloxymethylesterbromid

Eine Lösung von 10,71 g 3-Brommethyl-benzoesäure-pivaloyloxymethylester (erhalten gemäß Beispiel 3b) und 8,53 g Triphenylphosphin in 200 ml wasserfreiem Aceton wird unter Rühren und Feuchtigkeitsausschluß 4 Stunden lang zum Rückfluß erhitzt, wobei das sich bildende Phosphoniumsalz bereits auszufallen beginnt. Man läßt das Gemisch abkühlen, verdünnt mit 100 ml Ether, saugt das Reaktionsprodukt ab und wäscht mit Ether nach. Das Phosphoniumsalz wird bei 60° C im Vakuum über Phosphorpentoxid getrocknet. Das Produkt schmilzt bei 219° bis 220° C unter Zersetzung. Ausbeute: 17,16 g (= 89 % d. Th.). $^1$H-NMR ($CDCl_3$):
7,91 - 7,62 m 17H 5,62; 5,57 d 2H
7,37 - 7.30 m 2H 1,22 s 9H
5,86 s 2H

d) 3E-(m-Pivaloyloxymethyloxycarbonyl-benzyliden)-6β-(3′S-hydroxy-3′-cyclohexyl-1′E-propenyl)-7α-hydroxy-cis-bicyclo[3.3.0] octan

Zu einer Suspension von 9,17 g Natrium-bis-(trimethylsilyl)-amid in 300 ml Toluol in einem 500 ml Einhalskolben gibt man in einer Stick stoffatmosphäre bei Raumtemperatur unter Rühren rasch 29,58 g 3-Triphenylphosphoniummethyl-benzoesäure-pivaloyloxymethylester-bromid (hergestellt wie in Beispiel 3c beschrieben). Das Reaktionsgemisch nimmt sofort eine orangerote Färbung an. Man rührt zunächst 1 Stunde bei Raumtemperatur, erhitzt dann das Bad auf 80 - 90° C und hält 1 Stunde bei dieser Temperatur. Nach dem Abkühlen auf ca. 40° C fügt man 6,96 g 6β-(3′S-Hydroxy-3′-cyclohexyl-1′E-propenyl)-7α-hydroxycis-bicyclo-[3.3.0]octan-3-on hinzu und gibt noch soviel Toluol nach, daß der Flüssigkeitsspiegel bis zum Kolbenhals reicht. Das Gemisch wird zunächst einen Tag bei Raumtemperatur und dann weitere sechs Tage bei einer Badtemperatur von ca. 60° C gerührt. Nach dem Abkühlen gießt man das Gemisch in ca. 200 ml Wasser, schüttelt gut durch und trennt die organische Phase ab. Die wässrige Schicht wird zweimal mit je 100 ml Diethylether extrahiert. Die vereinigten organischen Phasen werden mit Natriumsulfat getrocknet und am Rotationsverdampfer eingeengt. Der Rückstand wird mit 40 ml Aceton versetzt, zum Abtrennen des gebildeten Triphenylphosphinoxids filtriert, und dann wird das Filtrat wieder eingedampft. Der Rückstand wird über Kieselgel mit Essigsäureethylester/Diethylether 1 : 4 chromatographiert. Aus dem erhaltenen EZ-Gemisch wird mittels präparativer "reversed phase" HPLC mit Methanol/Wasser 9 : 1 als Laufmittel die titelverbindung isoliert, die mit dem Produkt des Beispiels 2 identisch ist. Ausbeute: 2,99 g (= 47 % d. Th.).

## Ansprüche

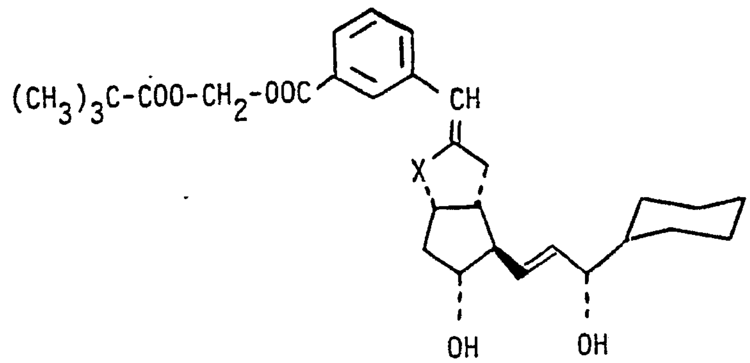

I

1) Neue Ester der Formel in der X für ein Sauerstoffatom oder für eine $CH_2$-Gruppe steht.

2) Als Ester gemäß Anspruch 1 das 2-Oxa-3Z-(m-pivaloyloxymethyloxycarbonyl-benzyliden)-6ß-(3′S-hydroxy-3′-cyclohexyl-1′E-propenyl)7α-hydroxy-cis-bicyclo[3.3.0]octan.

3) Als Ester gemäß Anspruch 1 das 3E-(m-Pivaloyloxymethyloxycarbonyl-benzyliden)-6β-(3′S-hydroxy-3′-cyclohexyl-1′E-propenyl)-7α-hydroxy-cis-bicyclo[3.3.0]octan.

4) Arzneimittel, dadurch gekennzeichnet, daß es als Wirkstoff einen Ester entsprechend Ansprüchen 1 - 3 enthält.

5) Arzneimittel gemäß Anspruch 4, dadurch gekennzeichnet, daß es als Wirkstoff pro Einzeldosis 0,01 - 50 mg eines Esters entsprechend Ansprüchen 1 - 3 enthält.

6) Arzneimittel gemäß Ansprüchen 4 oder 5, dadurch gekennzeichnet, daß darin der Ester entsprechend Ansprüchen 1 - 3 in Form einer Lipidemulsion oder in einer Liposomenform vorliegt.

7) Arzneimittel gemäß Ansprüchen 4 bis 6, dadurch gekennzeichnet, daß es zur parenteralen Applikation geeignet ist und als Wirkstoff pro Einzeldosis 0.01 bis 10 mg eines Esters entsprechend Ansprüchen 1 bis 3 enthält.

8) Arzneimittel gemäß Ansprüchen 4 oder 5, dadurch gekennzeichnet, daß es zur oralen Applikation geeignet ist und als Wirkstoff pro Einzeldosis 0.1 bis 50 mg eines Esters entsprechend Ansprüchen 1 -3 enthält.

9) Verfahren zur Herstellung von neuen Estern der Formel

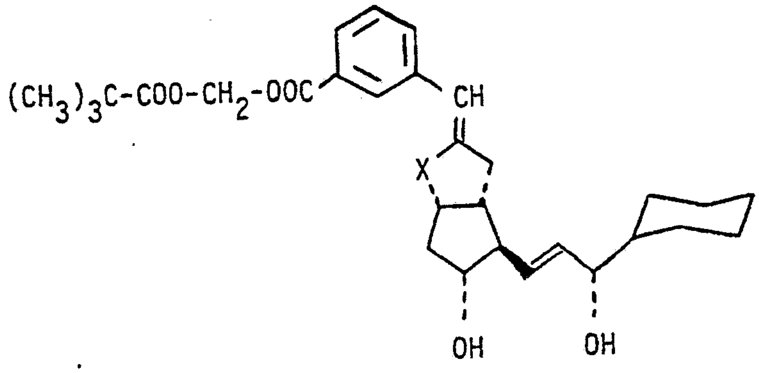

I

in der X für ein Sauerstoffatom oder für eine $CH_2$-Gruppe steht dadurch gekennzeichnet, daß man

a) eine Verbindung der Formel

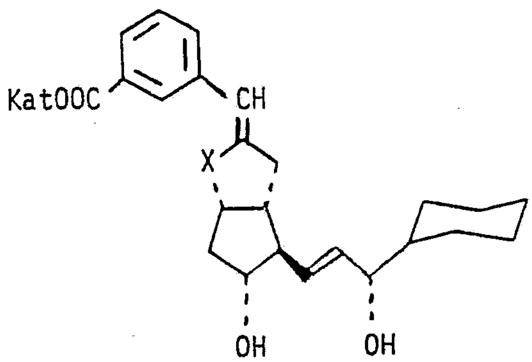

II

in der Kat ein Wasserstoffion oder ein Kation bedeutet und X die gleiche Bedeutung wie oben hat, gegebenenfalls nach intermediärem Schutz der Hydroxygruppen durch leicht wieder abspaltbare Gruppen, in einem inerten organischen Lösungsmittel gegebenenfalls unter Zusatz eines säurebindenden Stoffes und/oder unter Zusatz eines Alkalijodids mit einer Verbindung der Formel

$(CH_3)_3C\text{-}COO\text{-}CH_2\text{-}Y$ III

worin Y ein Chlor-, Brom- oder Jodatom oder eine Acyloxy-, Alkylsulfonyloxy- oder Arylsulfonyloxygruppe ist

umsetzt und gegebenenfalls die Schutzgruppen von den Hydroxygruppen wieder abspaltet, oder

b) eine Verbindung der Formel II unter den bei a) genannten Bedingungen mit einer Verbindung der Formel

$Cl\text{-}CH_2\text{-}Z$ IV

in der Z Brom, Jod oder einen Alkylsulfonyloxy- oder einen Arylsulfonyloxyrest bedeutet

zu einer Verbindung der Formel

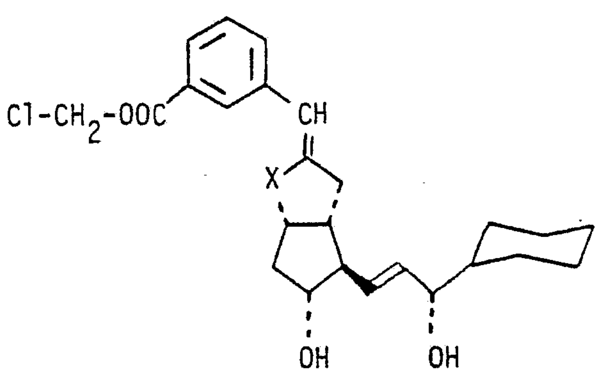

V

worin X die gleiche Bedeutung wie oben hat

umsetzt und dann diese unter den bei a) genannten Bedingungen mit einem Salz der Pivalinsäure zur

Reaktion bringt, und die gegebenenfalls eingeführten Hydroxy-Schutzgruppen abspaltet.

10) Verfahren gemäß Anspruch 9, dadurch gekennzeichnet, daß in der Verbindung der Formel III Y ein Chlor- oder Bromatom ist.

11) Verfahren gemäß Anspruch 9, dadurch gekennzeichnet, daß in der Verbindung der Formel III Y bzw. in der Verbindung der Formel IV Z eine Methylsulfonyloxy- oder eine p-Tolylsulfonyloxygruppe ist.

12) Verfahren zur Herstellung des 3E-(m-Pivaloyloxymethyloxycarbonylbenzyliden)-6β-(3′S-hydroxy-3′-cyclohexyl-1′E-propenyl)-7α-hydroxy-cis-bicyclo[3.3.0] octans entsprechend Anspruch 3, dadurch gekennzeichnet, daß man 3-Triphenylphosphoniummethyl-benzoesäure-pivaloyloxymethylesterbromid mit einer starken Base behandelt das dabei erhaltene Phosphinalkylenderivat mit 6β-(3′S-Hydroxy-3′-cyclohexyl-1′E-propenyl)-7α-hydroxy-cis-bicyclo-[3.3.0]octan3-on umsetzt und gegebenenfalls das E-Isomere aus dem angefallenen EZ-Gemisch in an sich bekannter Weise isoliert.

Patentansprüche für folgenden Vertragsstaat: ES

1) Verfahren zur Herstellung von neuen Estern der Formel

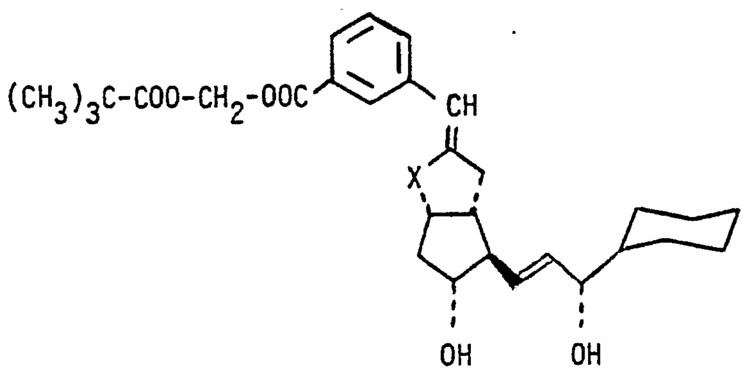

I

in der X für ein Sauerstoffatom oder für eine $CH_2$-Gruppe steht
dadurch gekennzeichnet, daß man

a) eine Verbindung der Formel

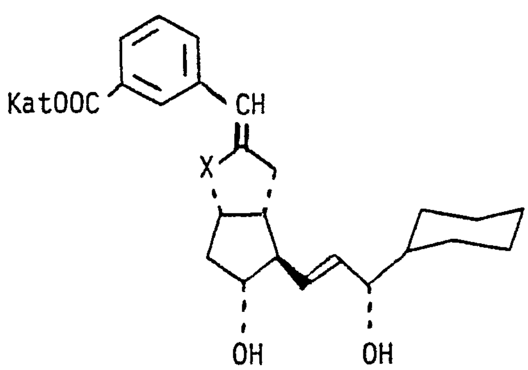

II

in der Kat ein Wasserstoffion oder ein Kation bedeutet und X die gleiche Bedeutung wie oben hat, gegebenenfalls nach intermediärem Schutz der Hydroxygruppen durch leicht wieder abspaltbare Gruppen, in einem inerten organischen Lösungsmittel gegebenenfalls unter Zusatz eines säurebindenden Stoffes und/oder unter Zusatz eines Alkalijodids mit einer Verbindung der Formel

$(CH_3)_3C\text{-}COO\text{-}CH_2\text{-}Y$ III

worin Y ein Chlor-, Brom- oder Jodatom oder eine Acyloxy-, Alkylsulfonyloxy- oder Arylsulfonyloxygruppe ist

umsetzt und gegebenenfalls die Schutzgruppen von den Hydroxygruppen wieder abspaltet, oder b) eine Verbindung der Formel II unter den bei a) genannten Bedingungen mit einer Verbindung der Formel

$Cl\text{-}CH_2\text{-}Z$ IV

in der Z Brom, Jod oder einen Alkylsulfonyloxy- oder einen Arylsulfonyloxyrest bedeutet zu einer Verbindung der Formel

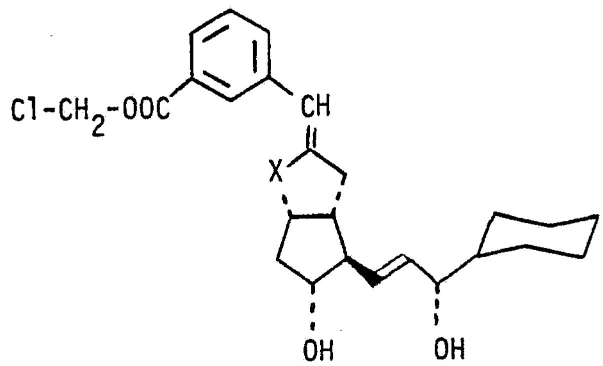

V

worin X die gleiche Bedeutung wie oben hat umsetzt und dann diese unter den bei a) genannten Bedingungen mit einem Salz der Pivalinsäure zur Reaktion bringt, und die gegebenenfalls eingeführten Hydroxy-Schutzgruppen abspaltet.

2) Verfahren gemäß Anspruch 1, dadurch gekennzeichnet, daß in der Verbindung der Formel III Y ein Chlor- oder Bromatom ist.

3) Verfahren gemäß Anspruch 1, dadurch gekennzeichnet, daß in der Verbindung der Formel III Y bzw. in der Verbindung der Formel IV Z eine Methylsulfonyloxy- oder eine p-Tolylsulfonyloxygruppe ist.

4) Verfahren zur Herstellung des 3E-(m-Pivaloyloxymethyloxycarbonylbenzyliden)-6β-(3′S-hydroxy-3′-cyclohexyl-1′E-propenyl)-7α-hydroxycis-bicyclo[3.3.0] octans als Verbindung der Formel I dadurch gekennzeichnet, daß man 3-Triphenylphosphoniummethyl-benzoesäure-pivaloyloxymethylesterbromid mit einer starken Base behandelt das dabei erhaltene Phosphinalkylenderivat mit 6β-(3′S-Hydroxy-3′-cyclohexyl-1′E-propenyl)-7α-hydroxy-cis-bicyclo-[3.3.0] octan3-on umsetzt und gegebenenfalls das E-Isomere aus dem angefallenen EZ-Gemisch in an sich bekannter Weise isoliert.

Patentansprüche für folgenden Vertragsstaat: GR

1) Neue Ester der Formel

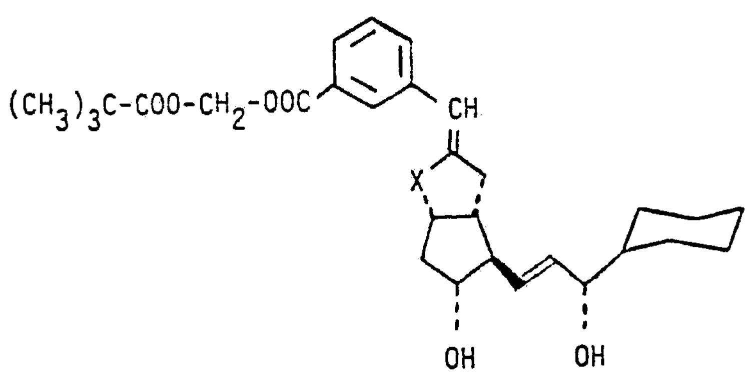

I

in der X für ein Sauerstoffatom oder für eine $CH_2$-Gruppe steht.

2) Als Ester gemäß Anspruch 1 das 2-Oxa-3Z-(m-pivaloyloxymethyloxycarbonyl-benzyliden)-6β-(3′S-hydroxy-3′-cyclohexyl-1′E-propenyl)-7α-hydroxy-cis-bicyclo[3.3.0] octan.

3) Als Ester gemäß Anspruch 1 das 3E-(m-Pivaloyloxymethyloxycarbonyl-benzyliden)-6β-(3′S-hydroxy-3′-cyclohexyl-1′E-propenyl)-7α-hydroxy-cis-bicyclo[3.3.0]octan.

4) Verfahren zur Herstellung von neuen Estern der Formel

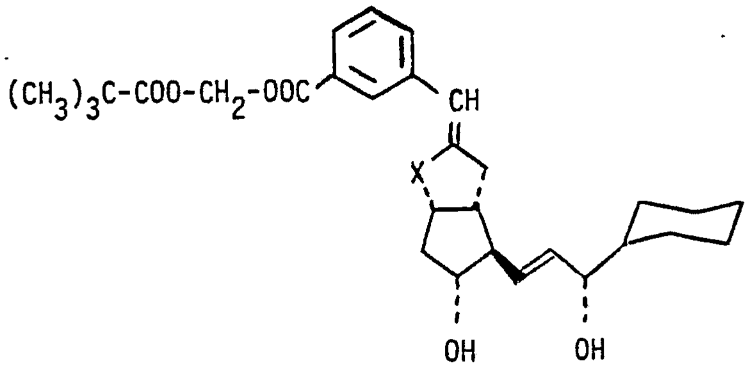

I

in der X für ein Sauerstoffatom oder für eine $CH_2$-Gruppe steht dadurch gekennzeichnet, daß man

a) eine Verbindung der Formel

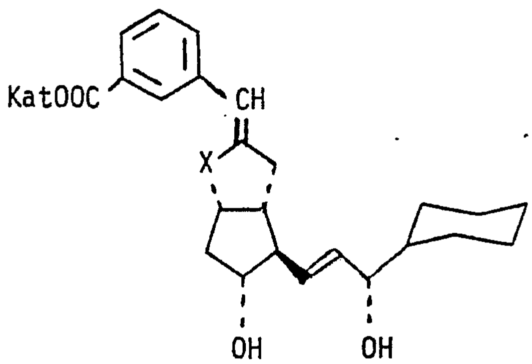

II

in der Kat ein Wasserstoffion oder ein Kation bedeutet und X die gleiche Bedeutung wie oben hat, gegebenenfalls nach intermediärem Schutz der Hydroxygruppen durch leicht wieder abspaltbare Gruppen, in einem inerten organischen Lösungsmittel gegebenenfalls unter Zusatz eines säurebindenden Stoffes und/oder unter Zusatz eines Alkalijodids mit einer Verbindung der Formel

$(CH_3)_3C\text{-}COO\text{-}CH_2\text{-}Y$ III

worin Y ein Chlor-, Brom- oder Jodatom oder eine Acyloxy-, Alkylsulfonyloxy- oder Arylsulfonyloxygruppe ist

umsetzt und gegebenenfalls die Schutzgruppen von den Hydroxygruppen wieder abspaltet, oder

b) eine Verbindung der Formel II unter den bei a) genannten Bedingungen mit einer Verbindung der Formel

$Cl\text{-}CH_2\text{-}Z$ IV

in der Z Brom, Jod oder einen Alkylsulfonyloxy- oder einen Arylsulfonyloxyrest bedeutet

zu einer Verbindung der Formel

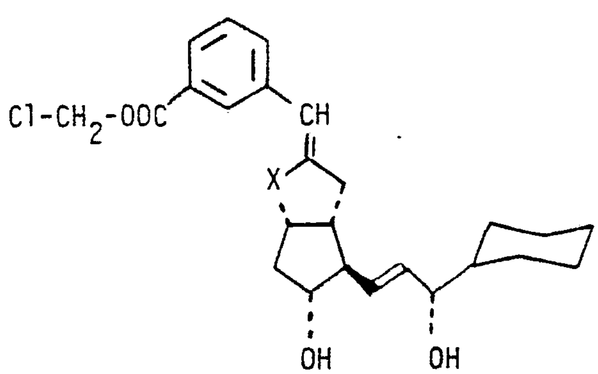

V

worin X die gleiche Bedeutung wie oben hat

umsetzt und dann diese unter den bei a) genannten Bedingungen mit einem Salz der Pivalinsäure zur

Reaktion bringt, und die gegebenenfalls eingeführten Hydroxy-Schutzgruppen abspaltet.

5) Verfahren gemäß Anspruch 4, dadurch gekennzeichnet, daß in der Verbindung der Formel III Y ein Chlor- oder Bromatom ist.

6) Verfahren gemäß Anspruch 4, dadurch gekennzeichnet, daß in der Verbindung der Formel III Y bzw. in der Verbindung der Formel IV Z eine Methylsulfonyloxy- oder eine p-Tolylsulfonyloxygruppe ist.

7) Verfahren zur Herstellung des 3E-(m-Pivaloyloxymethyloxycarbonylbenzyliden)-6β-(3′S-hydroxy-3′-cyclohexyl-1′E-propenyl)-7α-hydroxycis-bicyclo[3.3.0]octans entsprechend Anspruch 3, dadurch gekennzeichnet, daß man 3-Triphenylphosphoniummethyl-benzoesäure-pivaloyloxymethylesterbromid mit einer starken Base behandelt das dabei erhaltene Phosphinalkylenderivat mit 6β-(3′S-Hydroxy-3′-cyclohexyl-1′E-propenyl)-7α-hydroxy-cis-bicyclo-[3.3.0]octan-3-on umsetzt und gegebenenfalls das E-Isomere aus dem angefallenen EZ-Gemisch in an sich bekannter Weise isoliert.